# EUROPÄISCHE PATENTANMELDUNG

(11) **EP 0 927 553 A2**
(43) Veröffentlichungstag der Anmeldung: **07.07.1999**
(21) Anmeldenummer: 98123150.9
(22) Anmeldetag: 04.12.1998
(51) Int. Cl.: A61K 7/00

(54) **Mikroemulsionen**

(30) Priorität: 13.12.1997 DE 19755488
(71) Anmelder: Henkel KGaA, 40589 Düsseldorf (DE)
(72) Erfinder: Kahre, Jörg, Dr., 42799 Leichlingen (DE); Engels, Thomas, Dr., 50226 Frechen (DE); Hensen, Hermann, Dr., 42781 Haan (DE); Böttcher, Axel, 41569 Neuss (DE); Gutsche, Bernhard, Dr., 40724 Hilden (DE); Jackwerth, Bettina, 40764 Langenfeld (DE)

(57) **Zusammenfassung**

Vorgeschlagen werden Mikroemulsionen, enthaltend
(a) 5 bis 30 Gew.-% Ölkörper,
(b) 5 bis 80 Gew.-% anionische und/oder nichtionische Emulgatoren und
(c) 12 bis 30 Gew.-% Polyole
mit der Maßgabe, daß sich die Mengenangaben mit Wasser zu 100 Gew.-% ergänzen. Der besondere Vorteil der Emulsionen besteht darin, daß sie kalt herstellbar sind.

## Beschreibung

### Gebiet der Erfindung

Gegenstand der Erfindung sind Mikroemulsionen mit definierten Gehalten an Ölkörpern, Emulgatoren und Polyolen, ein Kaltverfahren zu ihrer Herstellung sowie die Verwendung der Mikroemulsionen zur Herstellung von kosmetischen bzw. pharmazeutischen Zubereitungen.

### Stand der Technik

Dusch- und Badeöle mit hohem Ölanteil sind seit einiger Zeit als pflegende und hautschonende Tensidpräparate im Markt. Da die Produkte hochkonzentriert sind und auch hochkonzentriert eingesetzt werden, sind sie schon von der Zusammensetzung her vergleichsweise teuer. Die Herstellkosten werden weiterhin dadurch erhöht, daß ihre stabile Herstellung bislang nur nach Heißverfahren erfolgen kann.

Aus der deutschen Offenlegungsschrift **DE-A1 4411557** (Henkel) sind in diesem Zusammenhang wäßrige Mikroemulsionen bekannt, die (a) 20 bis 60 Gew.-% eines Dialkylethers mit insgesamt 12 bis 24 Kohlenstoffatomen, (b) 20 bis 35 Gew.-% eines Ethylenoxidadduktes mit einem HLB-Wert von 6 bis 10, (c) 1 bis 10 Gew-% eines nichtionischen Ethylenoxidadduktes mit einem HLB-Wert oberhalb von 11 sowie (d) 1 bis 10 Gew.-% eines Diols oder Polyols enthalten. Gegenstand der internationalen Patentanmeldung **WO 97/23192** (Henkel) sind O/W-Mikroemulsionen, enthaltend (a) 30 bis 95 Gew.-% einer Ölphase bestehend aus Fettsäurealkylestern oder Triglyceriden, (b) Emulgatoren vom Typ der Alkyloligoglucoside und (c) Co-Emulgatoren vom Typ der Partialester von Fettsäuren mit Polyolen, die in einem zweistufigen Kaltverfahren hergestellt werden können. Des weiteren seien die Druckschriften **DE-A1 4337030, WO 93/11865 und EP-B1 0345586** (Henkel) genannt, aus denen PIT-Emulsionen bekannt sind, die Ölkörper, nichtionische Emulgatoren und Partialglyceride enthalten können.

Die Aufgabe der Erfindung hat daher darin bestanden, neue Dusch- und Badeöle mit hohem Ölanteil zur Verfügung zu stellen, die gleichzeitig über gute rückfettende Eigenschaften und hohe Lagerstabilität verfügen und sich zudem nach einem Kaltverfahren herstellen lassen.

### Beschreibung der Erfindung

Gegenstand der Erfindung sind Mikroemulsionen, enthaltend
(a) 5 bis 30, vorzugsweise 8 bis 12 Gew.-% Ölkörper,
(b) 5 bis 80, vorzugsweise 15 bis 70 Gew.-% anionische und/oder nichtionische Emulgatoren und
(c) 12 bis 30, vorzugsweise 14 bis 16 Gew.-% Polyole,
mit der Maßgabe, daß sich die Mengenangaben mit Wasser zu 100 Gew.-% ergänzen.

Überraschenderweise wurde gefunden, daß Mikroemulsionen, die die Komponenten (a) bis (c) in den genannten Mengen enthalten, ausgesprochen feinteilig sind, über ausgezeichnete pflegende und rückfettende Eigenschaften verfügen, auch bei Temperaturlagerung stabil sind und sich zudem auf kaltem Wege herstellen lassen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, Ester von C₆-C₂₂-Fettalkoholen und/oder Guerbetalkoholen mit aromatischen Carbonsäuren, vorzugsweise Benzoesäure, pflanzliche Öle, verzweigte primäre Alkohole, substituierte Cyclohexane, Dialkylcarbonate mit insgesamt 16 bis 32 Kohlenstoffatomen, Guerbetcarbonate, Dialkylether, Ringöffnungsprodukte von epoxidierten C₁₆-C₂₂-Fettsäureniedrigalkylestern mit Polyolen, insbesondere Trimethylolpropan und Pentaerythrit, und/oder aliphatische bzw. naphthenische Kohlenwasserstoffe in Betracht.

### Anionische Emulgatoren

Typische Beispiele für geeignete anionische Emulgatoren sind Alkylsulfate, vorzugsweise C₈-C₂₂-Fettalkohol- oder C₉-C₁₅-Oxoalkoholsulfate, Alkylethersulfate, vorzugsweise C₈-C₂₂-Fettalkohol- oder C₉-C₁₅-Oxoalkoholsulfate mit 2 bis 10 Mol Ethylenoxid in Form ihrer Natrium- oder Magnesiumsalze, Glycerinethersulfate, Fettsäureethersulfate, vorzugsweise Laurinsäure+1EO-sulfat, Hydroxymischethersulfate, Monoglycerid(ether)sulfate, vorzugsweise Kokosmono-glyceridsulfat, Fettsäureamid(ether)sulfate, Mono- und Dialkylsulfosuccinate, Mono- und Dialkylsulfosuccinamate, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

### Nichtionische Emulgatoren

Als nichtionische Emulgatoren kommen beispielsweise Vertreter aus mindestens einer der folgenden Gruppen in Frage:
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäurernono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagetungsprodukten von Ethylenoxid an Glycerin sind aus **DE-PS 2024051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE-OS 1943689, DE-OS 2036472** und **DE-A1 3001064** sowie **EP-A 0077167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

In einer bevorzugten Ausführungsform der Erfindung werden Emulgatorrnischungen von Alkylethersulfaten und Alkyloligoglucosiden im Gewichtsverhältnis 50 : 50 bis 95 : 45, vorzugsweise 80 : 20 bis 90 : 10 eingesetzt.

### Polyole

Polyole, die im Sinne der Erfindung in Betracht kommen, besitzen vorzugsweise 2 bis 15 Kohlenstoffatome und mindestens zwei Hydroxylgruppen. Typische Beispiele sind
- Alkandiole, vorzugsweise 1,2- bzw. α,ω-Alkandiole mit 5 bis 12 Kohlenstoffatomen, wie beispielsweise 1,2-Pentadiol, 1,5-Pentandiol, 1,2-Hexandiol, 1,6-Hexandiol, 1,2-Heptandiol, 1,7-Heptandiol, 1,2-Octandiol und 1,8-Octandiol, 12-Decandiol, 1,10-Decandiol, 1,2-Dodecandiol, 1,12-Dodecandiol sowie deren Gemische;
- Glycerin;
- Fettsäuremonoglyceride, vorzugsweise solche mit 6 bis 10 Kohlenstoffatomen im Acylrest;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10, wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Die erfindungsgemäßen Mikroemulsionen besitzen einen Wassergehalt von 1 bis 10, vorzugsweise 4 bis 8 Gew.-% Wasser.

### Gewerbliche Anwendbarkeit

Ein weiterer Gegenstand der Erfindung betrifft ein Verfahren zur Herstellung von Mikroemulsionen, bei dem man Mischungen, enthaltend
(a) 5 bis 30 Gew.-% Ölkörper,
(b) 5 bis 80 Gew.-% anionische und/oder nichtionische Emulgatoren und
(c) 12 bis 30 Gew.-% Polyole
mit der Maßgabe, daß sich die Mengenangaben mit Wasser zu 100 Gew.-% ergänzen, bei Temperaturen im Bereich von 15 bis 25°C vermischt.

Die erfindungsgemäßen Mikroemulsionen zeichnen sich durch ein besonders vorteilhaftes Rückfettungsvermögen aus. Ein weiterer Gegenstand der Erfindung betrifft daher ihre Verwendung zur Herstellung von Dusch- und Badeölen.

### Dusch- und Badeöle

In einer besonders einfachen Ausführungsform können die erfindungsgemäßen Mikroemulsionen unmittelbar als Dusch- und Badeöle verwendet werden. Sie können darüber hinaus jedoch weitere Hilfs- und Zusatzstoffe enthalten, wie beispielsweise Überfettungsmittel, Perlglanzwachse, Stabilisatoren, Konsistenzgeber, Verdickungsmittel, Kationpolymere, Siliconverbindungen, biogene Wirkstoffe, Konservierungsmittel, Hydrotrope, Solubilisatoren, Parfümöle, Farbstoffe und dergleichen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage : Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen; sowie deren Mischungen.

Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -di-ester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quaternierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor-, glucosid- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Amino-säuren, Ceramide, Pseudoceramide, essentielle Öle, Pflanzenextrakte und Vitaminkomplexe zu verstehen. Als **Konservierungsmittel** eignen sich beispielsweise Phenoxyethanol, Formaldehydlösung, Parabene, Pentandiol oder Sorbinsäure.

Als **Parfümöle** seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Sandel-, Guajak-, Zedem-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle kommen Ambroxan, Eugenol, Isoeugenol, Citronellal, Hydroxycitronellal, Geraniol, Citronellol, Geranylacetat, Citral, lonon und Methylionon in Betracht.

Als **Farbstoffe** können die für kosmetische Zwecke geeigneten und zugelassenen Substanzen verwendet werden, wie sie beispielsweise in der Publikation **"Kosmetische Farbemittel"** der Farbstoffkommission der Deutschen Forschungsgemeinschaff, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 5 Gew.-% - bezogen auf die Mikroemulsionen - betragen. Die Herstellung der Mittel kann durch übliche Kalt- oder Heißprozesse erfolgen; vorzugsweise arbeitet man nach der Phaseninversionstemperatur-Methode.

### Beispiele

In Tabelle 1 sind drei Beispielformulierungen für erfindungsgemäße Badeöle in Mikroemulsionsform wiedergegeben.

**Tabelle 1**

| **Badeöle in Mikroemulsionsform (Mengenangaben als Gew.-%)** | | | | |
|---|---|---|---|---|
| **Gruppe** | **Zusammensetzung** | **1** | **2** | **3** |
| Tenside | Sodium Laureth Sulfate | 16,0 | 16,0 | 16,0 |
| | Coco Glucosides | 5,0 | 5,0 | 5,0 |
| Ölkörper | Glyceryl Oleate | 10,0 | 10,0 | 10,0 |
| | Hexyldecanol | 2,2 | 2,2 | 2,2 |
| | Hexyldecanol Laurate | 2,2 | 2,2 | 2,2 |
| Polyol | 1,2-Hexandiol | 12,0 | - | - |
| | 1,6-Hexandiol | - | 12,0 | - |
| | Glyceryl Caprylate | - | 12,0 | 15,0 |
| Wasser | | ad 100 | | |

## Patentansprüche

1. Mikroemulsionen, enthaltend
(a) 5 bis 30 Gew.-% Ölkörper,
(b) 5 bis 80 Gew.-% anionische und/oder nichtionische Emulgatoren und
(c) 12 bis 30 Gew.-% Polyole
mit der Maßgabe, daß sich die Mengenangaben mit Wasser zu 100 Gew.-% ergänzen.

2. Mikroemulsionen nach Anspruch 1, **dadurch gekennzeichnet**, daß sie Ölkörper enthalten, die ausgewählt sind aus der Gruppe, die gebildet wird von Guerbetalkoholen auf Basis von Fettalkoholen mit 6 bis 18, vorzugsweise 8 bis 10 Kohlenstoffatomen, Estern von linearen C₆-C₂₀-Fettsäuren mit linearen C₆-C₂₀-Fettalkoholen, Estern von verzweigten C₆-C₁₃-Carbonsäuren mit linearen C₆-C₂₀-Fettalkoholen, Estern von linearen C₆-C₁₈-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, Estern von C₆-C₂₂-Fettalkoholen und/oder Guerbetalkoholen mit aromatischen Carbonsäuren pflanzlichen Ölen, verzweigten primären Alkoholen, substituierten Cyclohexanen, Dialkylcarbonaten, Guerbetcarbonaten, Dialkylethern, Ringöffnungsprodukten von epoxidierten C₁₆-C₂₂-Fettsäureniedrigalkylestern mit Polyolen und/oder aliphatischen bzw. naphthenischen Kohlenwasserstoffen.

3. Mikroemulsionen nach den Ansprüchen 1 und 2, **dadurch gekennzeichnet**, daß sie anionische Emulgatoren enthalten, die ausgewählt sind aus der Gruppe, die gebildet wird von Alkylsulfaten, Alkylethersulfaten, Glycerinethersulfaten, Hydroxymischethersulfaten, Monoglycerid(ether)sulfaten, Fettsäureamid(ether)sulfaten, Mono- und Dialkylsulfosuccinaten, Mono- und Dialkylsulfosuccinamaten, Ethercarbonsäuren und deren Salzen, Fettsäureisethionaten, Fettsäuresarcosinaten, Fettsäuretauriden, N-Acylaminosäuren, Alkyloligoglucosidsulfaten, Proteinfettsäurekondensaten und Alkyl(ether)phosphaten.

4. Mikroemulsionen nach den Ansprüchen 1 bis 3, **dadurch gekennzeichnet**, daß sie nichtionische Emulgatoren enthalten, die ausgewählt sind aus der Gruppe, die gebildet wird von Anlagerungsprodukten von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; C_{12/18}-Fettsäuremono- und -diestern von Anlagerungsprodukten von 1 bis 30 Mol Ethylenoxid an Glycerin; Glycerinmono- und -diestern und Sorbitanmono- und -diestern von gesättigten und ungesättigten Fettsäuren mit 6 bis 22 Kohlenstoffatomen und deren Ethylenoxidanlagerungsprodukten; Alkylmono- und -oligoglycosiden mit 8 bis 22 Kohlenstoffatomen im Alkylrest und deren ethoxylierte Analoga; Anlagerungsprodukten von 2 bis 15 bzw. 15 bis 60 Mol Ethylenoxid an Ricinusöl und/oder gehärtetes Ricinusöl; Polyolestern; Partialestern auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkoholen, Alkylglucosiden sowie Polyglucosiden; Trialkylphosphaten sowie Mono-, Di- und/oder Tri-PEG-alkylphosphaten; Wollwachsalkoholen; Polysiloxan-Polyalkyl-Polyether-Copolymeren; Mischestern aus Pentaerythrit, Fettsäuren, Citronensäure und Fettalkohol und/oder Mischester von Fettsäuren mit 6 bis 22 Kohlenstoffatomen, Methylglucose und Polyolen, sowie Polyalkylenglycolen.

5. Mikroemulsionen nach den Ansprüchen 1 bis 4, **dadurch gekennzeichnet**, daß sie als Emulgatoren Mischungen von Alkyl- und/oder Alkenylethersulfaten und Alkyl- und/oder Alkenyloligoglykosiden enthalten.

6. Mikroemulsionen nach den Ansprüchen 1 bis 5, **dadurch gekennzeichnet**, daß sie Polyole enthalten, ausgewählt aus der Gruppe, die gebildet wird von Alkandiolen, Glycerin, Fettsäuremonoglyceriden Alkylenglycolen, technischen Oligoglyceringemischen, Methylolverbindungen, Niedrigalkylglucosiden, Zuckeralkoholen, Zuckern und Aminozuckern.

7. Mikroemulsionen nach den Ansprüchen 1 bis 6, **dadurch gekennzeichnet**, daß sie als Polyole 1,2-bzw. α,ω-Alkandiole mit 5 bis 12 Kohlenstoffatomen und/oder Fettsäuremonoglyceride mit 12 bis 18 Kohlenstoffatomen im Acylrest enthalten.

8. Mikroemulsionen nach den Ansprüchen 1 bis 7, **dadurch gekennzeichnet**, daß sie 1 bis 10 Gew.-% Wasser enthalten.

9. Verfahren zur Herstellung von Mikroemulsionen, bei dem man Mischungen, enthaltend
(a) 5 bis 30 Gew.-% Ölkörper,
(b) 5 bis 80 Gew.-% anionische und/oder nichtionische Emulgatoren und
(c) 12 bis 30 Gew.-% Polyole
mit der Maßgabe, daß sich die Mengenangaben mit Wasser zu 100 Gew.-% ergänzen, bei Temperaturen im Bereich von 15 bis 25°C vermischt.

10. Verwendung von Mikroemulsionen nach den Ansprüchen 1 bis 8 zur Herstellung von Dusch- und Badeölen.
